# EUROPEAN PATENT APPLICATION

(11) **EP 1 762 258 A1**
(43) Date of publication of application: **14.03.2007**
(21) Application number: 06120286.7
(22) Date of filing: 07.09.2006
(51) Int. Cl.: A61M 5/14, A61M 5/168, G01F 23/26

(54) **Infusion apparatus with drip chamber having capacitance based liquid level monitoring**

(30) Priority: 08.09.2005 IT TO20050608
(71) Applicant: Hidex S.R.L., 10126 Torino (IT)
(72) Inventor: Pavese, Sergio, I-10126, TORINO (IT)
(74) Representative: Rondano, Davide

(57) **Abstract**

The infusion apparatus (I) comprises a reservoir (1) for an infusion liquid, a drip chamber (3) containing an infusion liquid amount with a level (L), an outflow tube (6b, 6c) that starts from the drip chamber (3) and ends with a cannula needle (2), and an outflow regulator (4) arranged along the outflow tube (6b, 6c) between the drip chamber (3) and the cannula needle (2). In order to detect variations in the liquid level (L) within the drip chamber (3) below a preset minimum value (Lmin) and/or above a preset maximum level (Lmax), the infusion apparatus (I) includes measure means (7, 8) for supplying measure data indicating variations in the dielectric constant of the assembly formed by the drip chamber (3) and by the amount of liquid contained therein upon variations in the liquid level (L). The measure means (7, 8) are arranged in particular to measure the capacitance between two electrodes (7) arranged on opposite sides relative to the drip chamber (3).

## Description

The invention relates to an infusion apparatus having the characteristics set forth in the preamble of claim 1.

An infusion apparatus generally comprises an infusion reservoir or infusion bag containing the infusion liquid and including an outflow port, a drip chamber connected through an upper inlet thereof to the infusion bag and through a lower outlet thereof to an outflow flexible tube ending with a cannula needle, and an outflow regulator interposed between the outlet of the drip chamber and the cannula needle. The infusion liquid contained in the infusion bag comes to the drip chamber through the upper inlet, is caused to drip within the chamber, in which an amount of infusion liquid is provided with a preset level which is lower than the maximum possible level, and then flows to the outflow tube through the lower outlet of the drip chamber. Means for detecting the level of the liquid amount are associated to the drip chamber and control means for signalling variations occurring in this level.

From US 6,337,631 it is known an infusion apparatus of the type specified above, to which an automatic monitoring instrument is associated, which comprises a clip intended to clamp a cylindrical tubular body of the drip chamber. The infusion apparatus is set such that the level of the liquid amount is kept constant within the drip chamber. To this end, a floater is provided within the drip chamber, which can rise and descend together with the level of liquid amount. A detecting unit has an emitter and a receiver being mounted on the side of the clip claws facing the floater. The drip chamber has transparent walls. The receiver is connected to an alarm signalling device by means of an electric wire. When the level of liquid amount within the drip chamber, and the floater therewith, descends below the level at which the emitter and receiver assembly is placed, and thus the floater does not prevent the signal from the emitter from being received by the receiver any longer, the alarm signalling device is activated and emits a sound, light (or other) as an alarm signal indicating that the infusion liquid is running out.

The apparatus known from US 6,337,631 solves efficiently the problem of monitoring a preset constant level of the liquid amount within the drip chamber, and avoids this control to be directly carried out by the staff. This known apparatus, however, has a first drawback due to the fact that the transmission of the signal emitted by the emitter not only occurs when the liquid level descends below the preset level, i.e. when the infusion liquid is running out, but also when due to a possible partial constriction and/or less absorption by the patient or a wrong setting of the doser or dropper, the liquid within the drip chamber exceeds the preset level. Furthermore, the level of the signal received by the receiver depends on the transparency of the infusion liquid, or rather on the transmission coefficient of the infusion liquid relative to the type of emitted signal. Moreover, it should be considered that a floater is required to be provided within the drip chamber, which implies a more complicated fabrication process if, for example, the drip chamber is made as one piece or as two pieces to be sealingly coupled to each other.

A further drawback is that the apparatus according to US 6,337,631 does not allow reading or providing indications, neither roughly, about the actual level of liquid amount within the drip chamber, as the information provided is limited to the fact that the liquid level has changed from what had been set as the reference level.

As the infusion apparatuses are disposable apparatuses, thus requiring to be as economical as possible, and to operate in the most reliable manner as possible, the solution according to US 6,337,631 is not completely satisfactory.

The object of the invention is therefore to improve an infusion apparatus with flow control which is less expensive and more reliable and further allows obtaining more information about the outflow conditions of the infusion liquid than in the prior art.

This object is fully achieved according to the invention by an apparatus of the type described above, which has the characteristics set forth in the characterizing portion of claim 1.

Further improvements of the invention are the object of the dependent claims.

In brief, the invention is based on the idea of using, as means for detecting the level of the infusion liquid amount within the drip chamber, means for measuring the dielectric constant of the assembly consisting of the infusion chamber and the infusion liquid amount contained therein. Particularly, these measuring means measure the capacitance of two electrodes that are arranged on opposite sides relative to the drip chamber.

The characteristics and advantages of the invention will appear from the detailed description below, which is merely given by way of a non-limiting example, with reference to the annexed drawings, in which:
Fig. 1 illustrates a schematic general view of an infusion apparatus according to a preferred embodiment of the invention;
Fig. 2 illustrates a variant embodiment of an infusion apparatus according to the invention, in which the control device of the infusion liquid flow controls, either by radio or by similar transmission means, at least one alarm signalling device, possible further alarm signalling devices being indicated with a dotted line;
Fig. 3 illustrates a variant embodiment of an infusion apparatus according to the invention, in which the alarm signalling device consists of a switch that can be controlled by the flow control device either by cable or by radio, and in which this controllable switch is inserted in parallel with the manual alarm-call push-button associated to a bed in a medical center or hospital;
Fig. 4 schematically illustrates an embodiment of the flow control device in the form of pincers or elastic clip, a battery and an electronic circuit being housed in the body thereof;

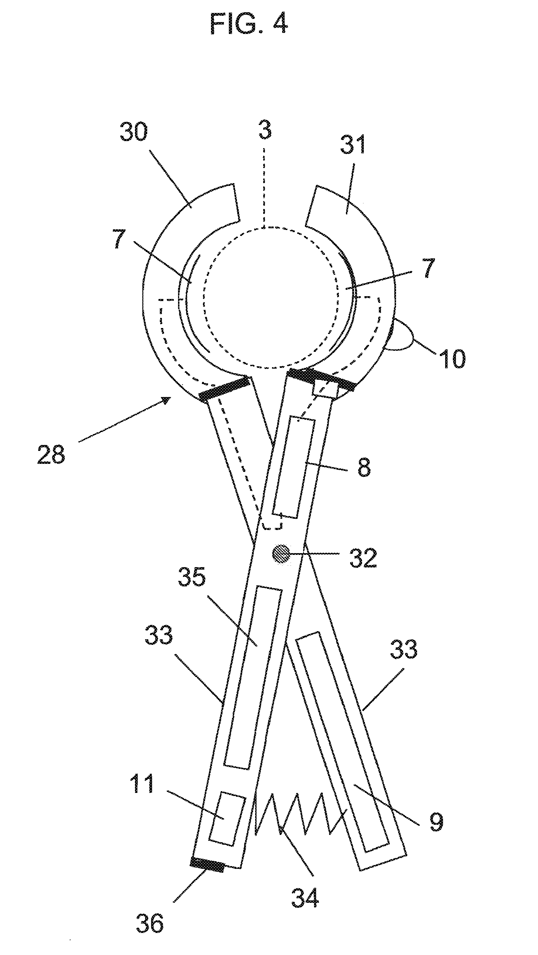

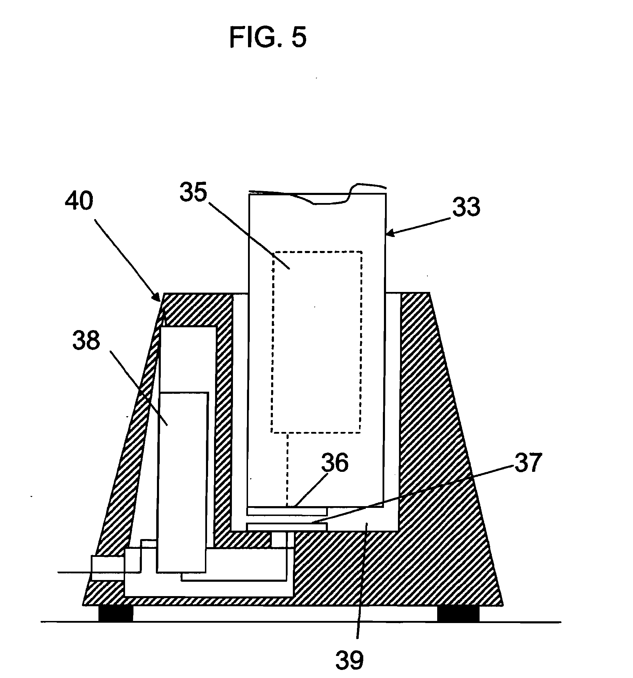

Fig. 5 illustrates a variant embodiment of the pincers or clip of Fig. 4, in which the pincers or clip, or any other support means for the electrodes, which houses therein at least a part of the electronic devices for measuring the capacitance between the electrodes and/or comparing and generating the control signal for the alarm signalling devices and at least one rechargeable battery, have outer contacts of the rechargeable battery matching electrical contacts supplying the recharge current, which are mounted in a housing shaped like a base or terminal block, and in which the base or terminal block also comprises a transformer/rectifier circuit that turns the network voltage into the recharge voltage for said at least one recharge battery;
Fig. 6 schematically illustrates the monitor of a central control unit provided with means for receiving and transmitting the capacity gauge signal and/or the alarm control signal either from or to several infusion apparatuses that are provided with the flow control device and positioned at various beds of a room in a medical center or hospital;
Fig. 7 illustrates a sectional view through an axial/diametral plane of an outflow regulator according to the present invention, in which the inlet part for the infusion liquid is made as one piece with the outlet end of the drip chamber;
Fig. 8 is a view similar to the one of Fig. 7 and illustrates a variant embodiment of the outflow regulator, in which the outflow regulator is sealingly applied by means of removable snap-coupling to the outlet end of the drip chamber;
Fig. 9 illustrates a top plan view (inlet side) of the outlet part of the outflow regulator;
Fig. 10 illustrates a further variant embodiment of the outflow regulator that can be adapted to any cylindrical drip chamber.

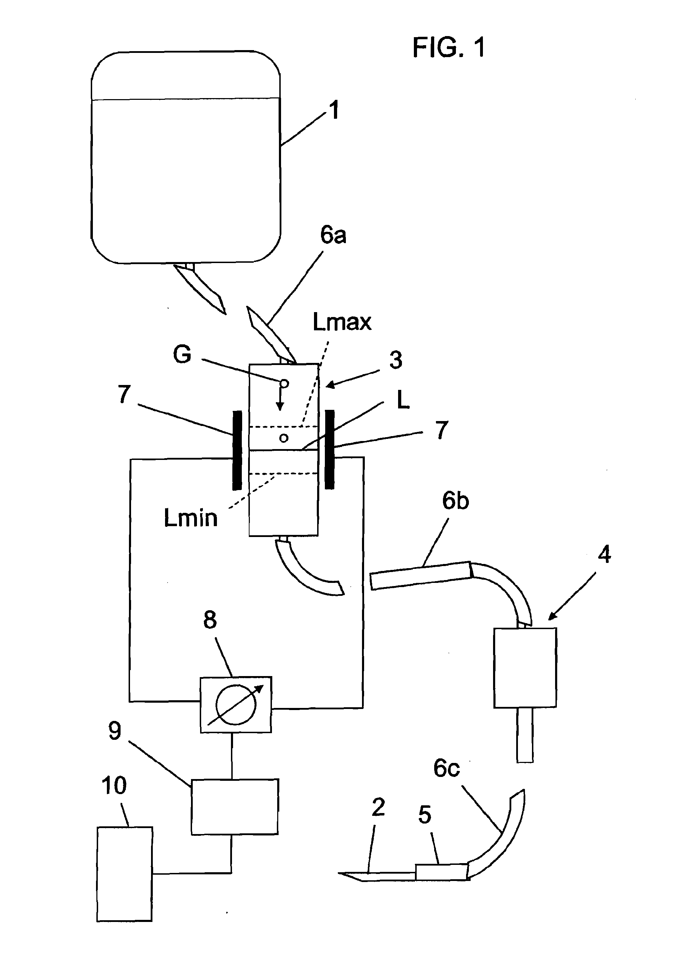

With reference to Fig. 1, an infusion apparatus comprises an infusion bag 1 containing an infusion liquid which must be intravenously administered to a patient by means of a cannula needle 2. A first section 6a of an outflow flexible tube connecting the outlet of the infusion bag 1 to the inlet of a drip chamber 3 branches off from the infusion bag 1.

The drip chamber 3 is filled to a preset level with a given amount of liquid. The liquid amount level, indicated L in Fig. 1, is an intermediate level between a maximum level corresponding to the condition of a fully filled drip chamber 3 and a zero level corresponding to the condition of a completely empty drip chamber 3. The infusion liquid is supplied to the drip chamber with a reduced flow rate, such that the infusion liquid flows by dripping into the liquid amount within the drip chamber 3, adjusting means being provided for adjusting the supply frequency of the infusion liquid drops G.

The outlet of the drip chamber 3 is connected by means of a second section 6b of flexible tube to an outflow regulator 4 by means of which the outflow rate of the infusion liquid to the cannula needle 2 can be adjusted. A third section 6c of flexible tube connects the outlet of the flow regulator 4 to a fitting 5 of the cannula needle 2.

A pair of electrodes 7, which are provided in the form of plates of electrically conductive material, are arranged outside the drip chamber, on diametrally opposite sides relative thereto, and are substantially centered in a vertical direction relative to the liquid amount level L within the drip chamber 3. The opposed electrodes 7, the drip chamber 3 and the liquid contained therein form an electrical capacitor. As the liquid amount level L within the drip chamber 3 is variable, the dielectric material of the capacitor, which consists of the drip chamber 3 and the liquid contained therein, has a correspondingly variable dielectric constant. Since the capacitance of the capacitor changes proportionally to the dielectric constant, the liquid amount level L within the drip chamber 3 can be measured by measuring the capacitance of the capacitor formed by the opposed electrodes 7, the drip chamber 3 and the liquid contained therein.

This measurement is continuous, i.e. the variations in the capacity as a function of variations of level are continuously detected throughout the extension of the electrodes 7 in the direction of the level variation, i.e. in a direction substantially perpendicular to the surface of the liquid amount within the drip chamber 3.

In a simplified embodiment, after a maximum level Lmax and a minimum level Lmin have been set, which are designated with a dotted line in Fig. 1, the capacitance values corresponding to the maximum and minimum levels can be determined, and these capacitance values can thus be set as threshold values for the generation of control signals intended to activate alarm signalling devices. These control signals are, in this case, generated when the measured capacitance value descends below the threshold value related to the minimum level Lmin and/or rises above the threshold value related to the maximum level Lmax.

In Fig. 1 a block diagram is illustrated, in which a capacitance measuring unit is indicated 8, a control signal generating unit is indicated 9, and an alarm signalling device is indicated 10, which device can be activated by the control signal generated by the control signal generating unit 9.

The capacitance measuring unit 8 can be provided, in a manner known per se by those skilled in the art, as an electronic unit of any type, which will not be described herein in detail.

The control signal generating unit 9 is also well known to those skilled in the art in various forms, and advantageously comprises a comparison section for comparing the measure signal supplied from the capacitance measuring unit 8 with the threshold values corresponding to the maximum level Lmax and minimum level Lmin of the liquid within the drip chamber 3. Based on the signal outputted from the comparison section, a generating section of the control signal generating unit 9 generates the control signal and transmits it to the alarm signalling device 10. Instead of an alarm signalling device 10, a number of devices can be provided which are capable of generating different types of alarm, both sound and visual.

The apparatus according to the invention is very flexible in terms of both physical and operating configurations. Depending on the requirements and economic resources, the number of functions that the apparatus is capable of carrying out can be either increased or decreased.

Generally, the measure signal contains further information in addition to the mere instant value of liquid level L within the drip chamber 3. In fact, as the measurement is continuous, the variation in the liquid level L within the drip chamber 3 can be further periodically read and stored in a special memory, and thus the time trend and the rate of this variation can be established by providing a digital implementation of the capacitance measuring unit 8 and/or of the control signal generating unit 9 and/or of the alarm signalling devices 10, for example in the form of a microprocessor circuit and the like. This can be advantageous, for example, in order to start the alarm signal before the maximum level threshold Lmax or the minimum level threshold Lmin is actually exceeded, based for example on the time trend of the liquid level variation in either direction. The real-time assessment of the variation in the liquid level L within the drip chamber 3 makes it possible also to adjust the dripping speed before an alarm condition occurs.

The control signal transmission to the alarm signalling devices 10 can be carried out, rather than by cable, by means of radio frequency signal, or the like, which avoids laying cables and allows making the infusion apparatus communicate with a number of alarm signalling devices 10 that are located in different places and diversified according to the type of alarm signal generated.

A further advantageous characteristic is that a memory intended to store a univocal identification code can be associated with the control signal generating unit 9. The use of a univocal identification code allows enabling the communication, and thus receiving the control signals and/or identifying as valid the control signals generated by an infusion apparatus, only with one or more specific alarm signalling devices. The identification code of the infusion apparatus and of the alarm signalling devices associated therewith can be thus univocally related to a station, such as a bed at which the infusion apparatus is installed.

When an electronic control unit is provided as the alarm signalling device, on the other hand, then the identification code of the control signal generating unit univocally identifies which infusion apparatus is in the alarm condition, and based for example on a list stored in the electronic control unit, allows indicating the exact location of the infusion apparatus from which the control signal has started.

When the control signal is transmitted by radio, or the like, a central control unit can be also provided, which can be for example placed in a surveillance station. The central control unit can be provided with identification means suitable to identify, due to the identification code, which apparatus has generated the control signal, as well as with display means allowing the identification code to be associated with a localization layout of the bed, either graphically or alphanumerically, in such a manner that the station at which the alarm has been generated can be immediately detected.

By generating a radio signal or the like, which is provided with a univocal identification code of the station that has produced the alarm signal, more than one alarm signalling device can be also registered as the alarm signalling device being univocally related to a given station. One or more alarm signalling devices can be also registered for different positions, in such a manner that both a general alarm warning, which is activated irrespective of the station, and alarm warnings specific for each station are available.

Several possible variant embodiments of the infusion apparatus according to the invention will be described herein after.

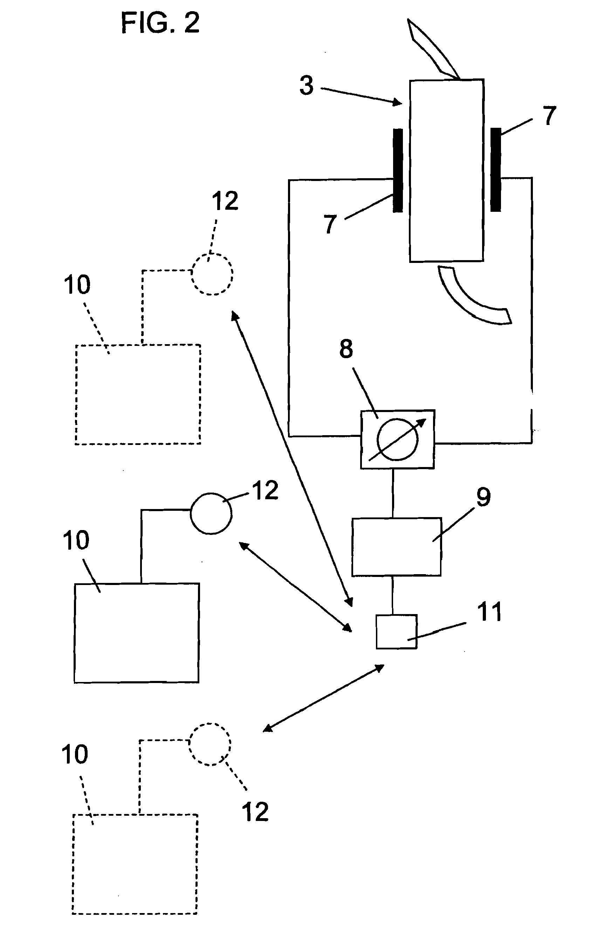

With reference to Fig. 2, according to a variant embodiment, the infusion apparatus comprises a transmitter 11 for transmitting the control signal by radio, whereas the alarm signalling devices 10 are each provided with a respective receiver 12, by means of which they receive the control signal transmitted by the transmitter 11 by radio.

The transmitter 11 and the receiver 12 can also be provided otherwise. As the supply from a power network does not imply particular drawbacks for the alarm signalling devices 10, while, on the other hand, it can be uncomfortable or hamper the capacitance measuring unit 8 and the control signal generating unit 9 on the infusion apparatus because of the supply cables necessarily provided thereon, an advantageous embodiment provides that the transmitter 11 associated with the control signal generating unit 9 consists of a transponder device. This device is known and widely used.

In this case, a transmitter/receiver 12 associated with the alarm signalling devices 10 periodically queries the transponder connected to the control signal generating unit 9 on the infusion apparatus. The transponder applies the information relating to the measurement of the capacity and/or to the control signal in the form of modulation of the carrier received and uses the power of this carrier to transmit the signal back to the transmitter/receiver 12 associated with the alarm signalling devices 10 from which the query signal has started. This solution allows a considerable reduction in the consumption of the units provided for the measurement of the capacitance and for the generation and transmission of the control signal. These units can be thus supplied for a long time by means of a small battery, without requiring any cable supply from the power network.

In Fig. 2, further alarm signalling devices 10 are illustrated with a dotted line, each of which can be univocally associated with an infusion apparatus due to the identification code of the infusion apparatus that is also transmitted along with the control signal. The alarm signalling devices 10 can differ both in location and in type of alarm being generated (for example, visual and/or sound, etc.).

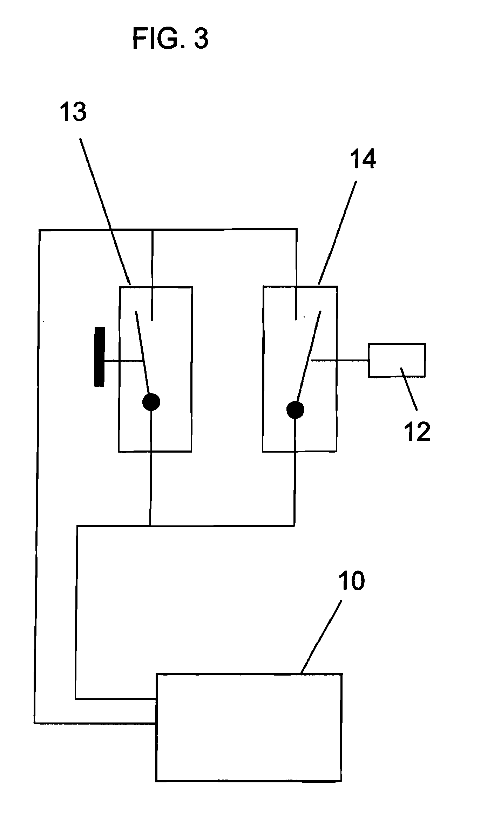

Fig. 3 illustrates a variant embodiment, which is particularly advantageous since it provides using alarm signalling devices that are already normally provided on stations (beds) in medical centres or hospitals. Generally, at least one push-button (indicated 13 in Fig. 3) for calling the medical staff is associated with each bed in a medical center or hospital. This push-button can be manually activated to close a control circuit that activates the alarm signalling device 10, which is generally of a visual and/or sound type and can also provide an indication on which station has sent the command. Even in this case, the alarm signalling device 10 can actually consist of several different devices remotely placed relative to the station where the infusion apparatus is installed.

According to the variant embodiment of Fig. 3, a switch 14 is provided, which is capable of being controlled by the control signal that is supplied, either by radio or by cable, by the control signal generating unit 9 associated with the infusion apparatus. The switch 14 is inserted in the control circuit that activates the alarm signalling device 10 so as to bypass the call push-button 13, generally in parallel to the latter. This solution is particularly advantageous in terms of costs, since the alarm signalling circuits residing in the medical centres and hospitals are generally already provided with an indication of the station from which the alarm has been generated. This solution is also particularly advantageous in terms of construction, since the switch 14 can be easily integrated in a housing box that is either mounted to or enclosed in a wall. The so-called mounting boxes currently available on the market have a sufficient volume for housing these devices. Furthermore, when the switch 14 is connected by cable, the connection can be made by means of a female connector which is structurally associated with the switch and in which a male connector can be fitted, which is mounted at an end of the connecting cable. Moreover, the solution in Fig. 3 does not require using further alarm signalling devices, or long and expensive installation activities.

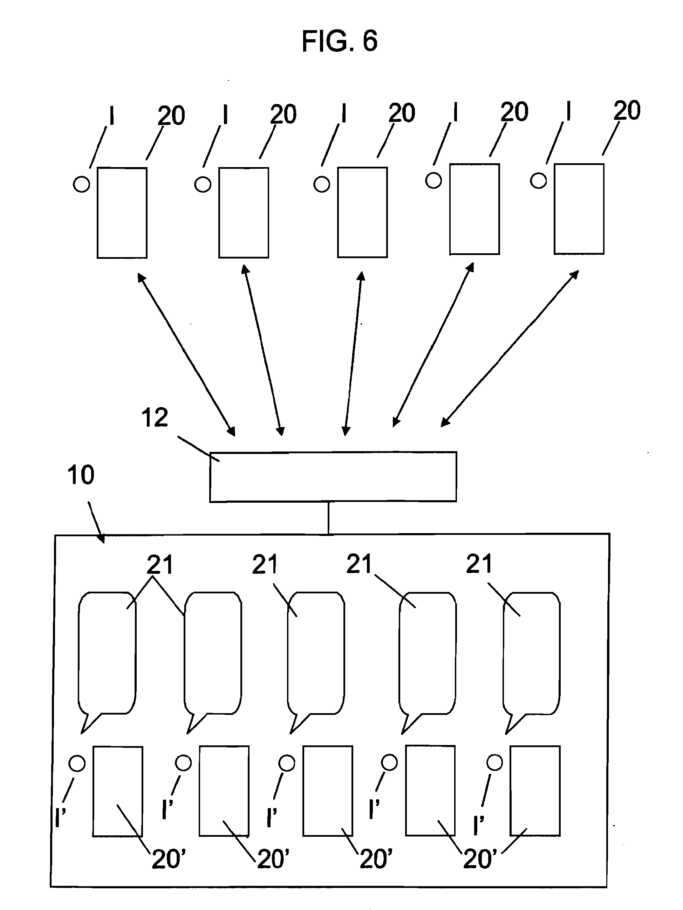

Fig. 6 schematically shows a more complicated system, in which several beds 20 are provided in a hospital room, each of which being associated with an infusion apparatus (generally indicated I) according to the invention, particularly according to the variant embodiment of Fig. 2.

In this case, each infusion apparatus I is univocally marked by an identification code, which identifies both the apparatus and the associated bed. The alarm signalling device 10 is embodied, in this case, by a computer screen on which images or icons 20' are displayed, which represent the beds 20 according to their actual space layout, as well as images or icons I' which represent the infusion apparatuses I associated with each bed 20.

A different colour, or simply the absence of an image or icon I', can signal the absence or inactivity of the corresponding infusion apparatus. All the infusion apparatuses shown in Fig. 6 result to be active.

The computer supervising the operation of the infusion apparatuses I is provided with a receiving unit 12, that can also operate as a transmission unit, by means of which the control signals generated by the different infusion apparatuses I are received. The identification code of the corresponding infusion apparatus I is associated with each control signal, and thus each control signal is univocally related by the computer to the corresponding bed 20, i.e. to the corresponding image or icon 20'. Furthermore, based on the control signals received by the receiving unit 12 (possibly after a periodical query of the individual infusion apparatuses I, in the case of transmitting/receiving units), the operating parameters of each infusion apparatus I are displayed on the computer screen 10 in an area or block 21 and are univocally related to the image or icon I' of the corresponding apparatus.

Different alternative operating modes can be provided within the control system of Fig. 6. For example, the periodical query of the various infusion apparatuses can be not limited to the detection of an alarm condition, rather it may provide for the acquisition of the data relative to the measurement of the liquid level L in the drip chamber. These data can be then stored in the computer and displayed in the area 21 of the screen 10, which is associated with each individual infusion apparatus. Furthermore, the computer can calculate the speed of the liquid level variation and thus display an alarm signal before the liquid level actually reaches the alarm threshold.

It should be noted that in the control system according to Fig. 6, the control signal generating unit 9 does not have to be necessarily integrated within the infusion apparatus, but can be formed by the same computer supervising the operation of the infusion apparatuses. In this case, only a capacitance measure unit, an identification code setting unit, and a transmitting/receiving unit will be provided for each infusion apparatus. This allows reducing both the overall size of the electronic circuits to be installed in the infusion apparatus and the consumption of electric power.

From the constructive point of view, the infusion apparatus according to the present invention can be made in various ways.

According to a first solution, the electrodes 7 are mounted on the peripheral walls of the drip chamber 3. In this case, the electrodes 7 can be made as plates or sheets of electrically conductive material which are attached to the peripheral walls of the drip chamber either in a fixed or removable manner, for example fitted in suitable seats formed by the walls of the drip chamber 3, and/or joined by means of an adhesive layer. When the drip chamber 3 has a curved peripheral wall, particularly a cylindrical wall, the plates or metal sheets forming the electrodes 7 can be pre-shaped to match the curvature of the wall portion of the drip chamber 3 on which they have to be attached.

Also the batteries and electronic devices, such as the capacitance measuring unit 8, the control signal generating unit 9 and the alarm signalling device 10 can be housed, either in a fixed or removable manner, within a suitable extension of the drip chamber 3.

Of course, this first solution is quite unsuitable to the existing drip chambers. It is thus preferred that the electrodes 7 and electronic devices such as the capacitance measuring unit 8, the control signal generating unit 9 and the alarm signalling device 10 are mounted in a pincer or clip member. This second solution is schematically illustrated in Fig. 4, in connection with the case of a common drip chamber shaped as a circular-section cylinder.

The pincer or clip member, generally indicated 28, has two claws 30 and 31 having the shape of annular segments, which encompass the drip chamber 3 on diametrally opposite sides. A respective electrode 7 made as a plate or sheet of an electrically conductive material having a correspondingly curved shape is attached to the inner side of each claw 30, 31.

The two claws 30 and 31 extend from the free ends of a pair of straight rods 33 which are hinged to each other in a hinge axis 32 parallel to the axis of the drip chamber 3. The end portions of the rods 33 opposite the claws 30 and 31 relative to the hinge axis 32 act as grip portions. A spring 34 or other equivalent elastic means are provided between the two claws 30 and 31 and tend to move the two claws close to each other and thus to clamp the drip chamber 3 therebetween.

The pincer or clip member 28 is made such as to form the seats for a supply battery 35, for the capacitance measuring unit 8, for the control signal generating unit 9, for the alarm signalling device 10, respectively, the latter being provided, in this case, as a light signaller (such as a LED), and possibly also for the transmitter 11 (or the transmitter/receiver 12). As the functions carried out by said electronic devices are basically simple, these devices can be small-sized and thus integrated within the pincer or clip member 28 without the consequent increase in the overall size of the latter impairing the manoeuvrability and usefulness thereof.

This second solution is particularly advantageous as it can be easily applied to any drip chamber, provided that the variation in the liquid level within the drip chamber occurs in the axial direction of the chamber, and the radial size of the chamber is not excessively large. Furthermore, as the physical magnitude being measured is the capacitance between the electrodes 7, which varies based on the variation in the dielectric constant of the drip chamber 3 due to the variation in the liquid level, the pincer or clip member 28 can be also applied to drip chambers having a non-circular section and/or not transparent.

According to a further characteristic of the invention, the supply battery 35 can be of a non-rechargeable type, in which case the relative seat must be openable from the outside for battery replacement.

Alternatively, the supply battery 35 can be of a rechargeable type. In this case, the pincer or clip member 28 advantageously has at least one connector accessible from the outside for connecting the rechargeable battery to a recharge power supply.

As shown in Fig. 4 and 5, the pincer or clip member 28 can be provided, in place of the connector, with contacts 36 for connection to corresponding contacts 37 of a recharge power supply 38. In this case, the recharge power supply 38 can be provided in the form of a terminal block, as normally occurs for cellular or wireless phones. Particularly, in the embodiment of Fig. 4, the contacts 36 are provided on the head end of one of the rods 33 of the pincer or clip member 28, whereas the contacts 37 are provided on the bottom of a seat notch 39 formed in a support and recharge base 40, in which the recharge power supply 38 can also be housed.

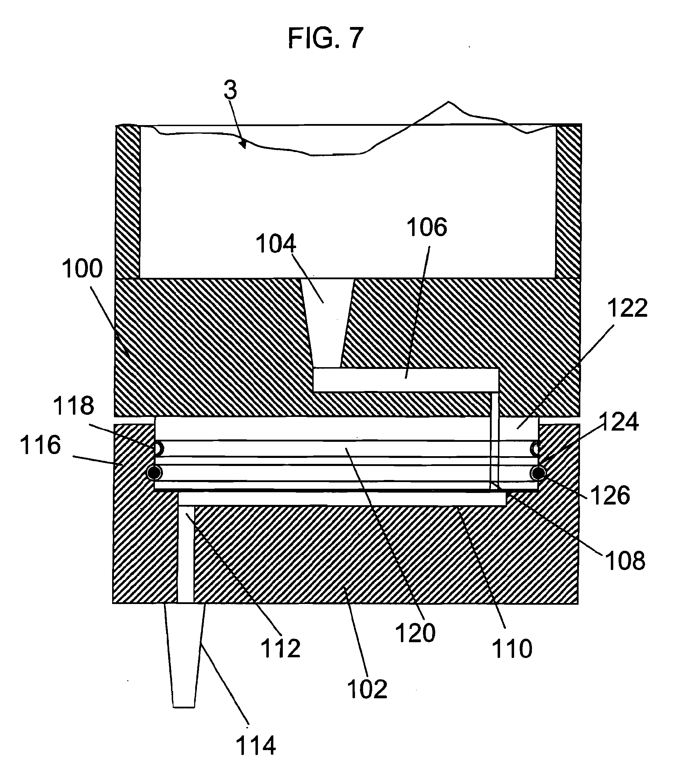

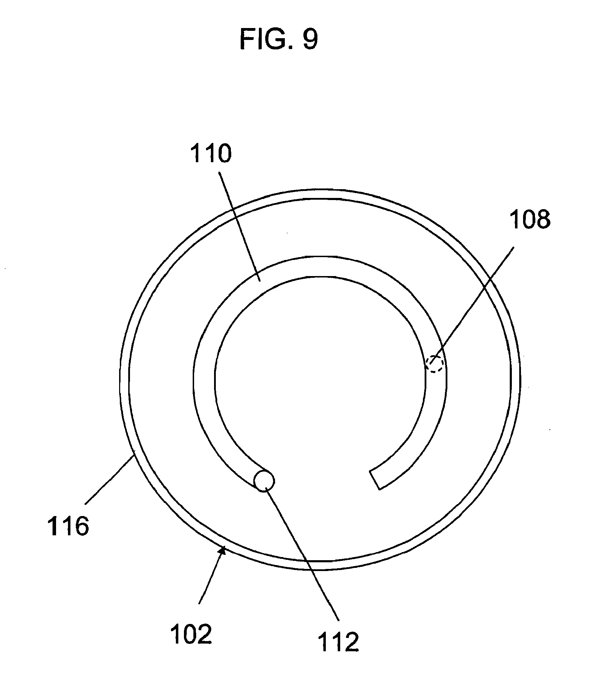

Fig. 7 to 10 illustrate different variant embodiments of the outflow regulator 4 according to the present invention. For a better use of the characteristics of versatility and measurement accuracy of system for detecting the variation in the liquid level within the drip chamber 3, it is advantageous to provide an outflow regulator, which has an improved flow-regulation capacity as compared to the known devices, particularly in terms of precision and repetitiveness of regulation.

According to the variant embodiments illustrated, the outflow regulator 4 can be directly mounted to the outlet of the drip chamber 3, or made at least partially as one piece with the part of the drip chamber 3 in which the outlet is provided.

The outflow regulator 4 shown in Fig. 7 is of the type partially made as one piece with the outlet end of the drip chamber 3. In this case, the outflow regulator 4 substantially consists of an upper inlet part 100 and a lower outlet part 102, which are sealingly engaged with each other and can be displaced relative to each other along a preset path. The inlet part 100 is made as one piece with, or forms the outlet end of the drip chamber 3 and has an inlet opening 104 through which the infusion liquid contained in the drip chamber 3 flows in. The inlet opening 104 is connected by means of an inner duct 106 to an outlet hole 108 provided on the lower coupling side of the inlet part 100 to the outlet part 102. The outlet part 102 has on its upper side, facing the inlet part 100, a collection groove 110 in which the infusion liquid coming from the outlet hole 104 of the first inlet part 100 is collected. The collection groove 110 extends parallel to the relative displacement path of the two parts 100 and 102 of the outflow regulator and is arranged in a position matching the outlet hole 108 of the inlet part 100 throughout said relative displacement path. An outlet channel 112 opens at an end of the collection groove 110, which channel passes through the outlet part 102 and comes to a delivery outlet 114 of the outflow regulator 4 provided on the lower side of the outlet part 102.

The outlet hole 108 of the inlet part 100 and the collection groove 110 of the outlet part 102 of the outflow regulator have a diameter and a cross-section, respectively, such that the outflow resistance is significatively affected by the capillary forces. In this way, upon relative displacement between the two inlet and outlet parts 100 and 102, the distance between the outlet hole 404 of the inlet part 100 and the outlet channel 112 placed at one of the ends of the collection groove 110 is changed. The outflow resistance, and accordingly the outflow rate, of the infusion liquid from the drip chamber 3 is thus changed.

Advantageously, the two inlet and outlet parts 100 and 102 of the outflow regulator 4 are made in the form of a circular-base cylinder and sealingly engage with each other by means of snap-fitting means so as to be capable of rotating about the central axis of the cylinder. The outlet hole 108 of the inlet part 100 is provided in an eccentric position, while the collection groove 110 extends on a circular path having a radius equal to the eccentricity of the outlet hole 108. Furthermore, the collection groove 110 extends less than 360° in order for the end at which the outlet channel 112 is provided and the opposite closed end to be held spaced from each other.

The snap-engaging means which allow the rotation and ensure the sealing between the inlet part 100 and the outlet part 202 of the outflow regulator consist, in the embodiment in Fig. 7, of a continuous circular ring 116 coaxially extending from the outlet part 102 to the inlet part 100. The ring 116 is made of a resiliently compliant material and forms on its inner peripheral surface an annular, preferably rounded projection 118, which extends radially inwards and is arranged at an annular groove 120 provided on the outer peripheral surface of a cylindrical coupling extension 122 of the inlet part 100. The outer diameter of the cylindrical extension 122 corresponds to the inner diameter of the ring 116. The ring 116 and the cylindrical extension 122 may have two opposite half-grooves 124 in which a sealing gasket 126, such as an O-ring, or the like, is received.

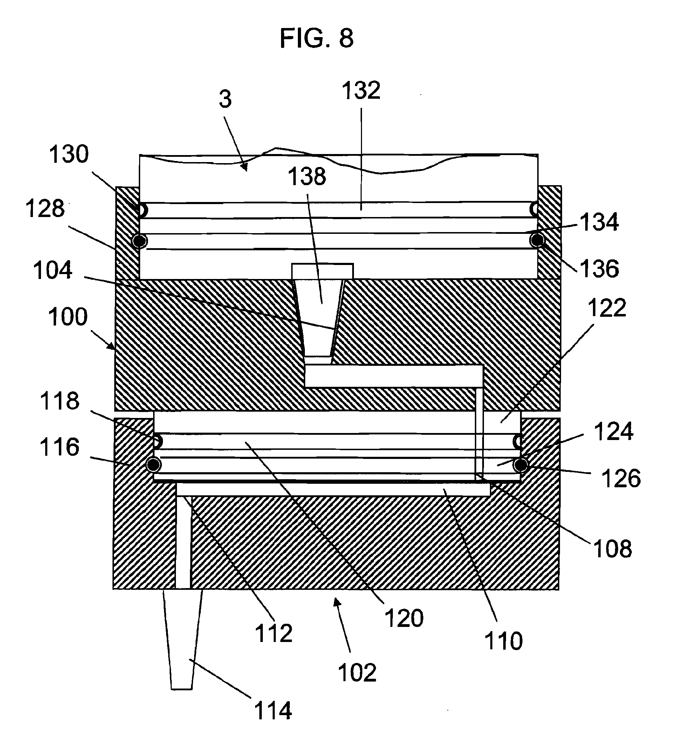

The outflow regulator 4, particularly the inlet part 100, can be provided such as to be removably coupled to the outlet end of the drip chamber 3. This variant embodiment is illustrated in Fig. 8, in which the same numerals designate parts and elements that are either identical or correspond to those of Fig. 7.

According to this variant embodiment, the inlet side of the inlet part 100 of the outflow regulator 4 and the outlet end of the drip chamber 3 are provided with respective complementary-shaped coupling means and snap-locking means by means of which a single integrated device consisting of the drip chamber 3 and the outflow regulator 4 can be formed.

In the example of Fig. 8, the inlet end (upper end) of the inlet part 100 is cup-shaped and has a peripheral ring 128 forming on the inner side surface thereof an annular projection 130 radially extending inwards and engaging in a matching annular groove 132 provided in the lower end portion of the drip chamber 3. As for the two parts 100 and 102 of the outflow regulator 4, also in this case, the peripheral ring 128 of the inlet part 100 and the lower end portion of the drip chamber 3 can have two opposite half-grooves 134 in which a sealing gasket 136, such as an O-ring, or the like, is received.

The inlet opening 104 provided in the inlet part 100 houses, in this case, an outlet spout 138 of the drip chamber 3. The sealing between the spout 138 and the inlet opening 104 is achieved by form-fitting and/or by means of suitable gaskets (not shown).

With the structure described above, the outflow regulator 4 is easy to operate and ensures a high precision, which allows the user to set the outflow rate in an easy and repeatable manner.

For example, by providing reference scales on the two parts of the outflow regulator, and when the viscosity of the infusion liquid is indicatively known, the outflow regulator can be properly set from the beginning, within small tolerances, thus dramatically reducing the need of monitoring or correcting the set regulation later.

The outflow regulator according to the present invention also allows applying motor-driven means for controlling the adjustment displacement. These motor-driven means consist of, for example, a small electric motor that is coupled to one of the parts of the flow regulator and that can be remotely controlled both manually and automatically based on the data provided by the flow control device associated with the drip chamber. In this second case, the automatic adjustment can be carried out both directly by the flow control device and remotely by a surveillance electronic unit communicating with the flow control device and with the electric motor driving the outflow regulator.

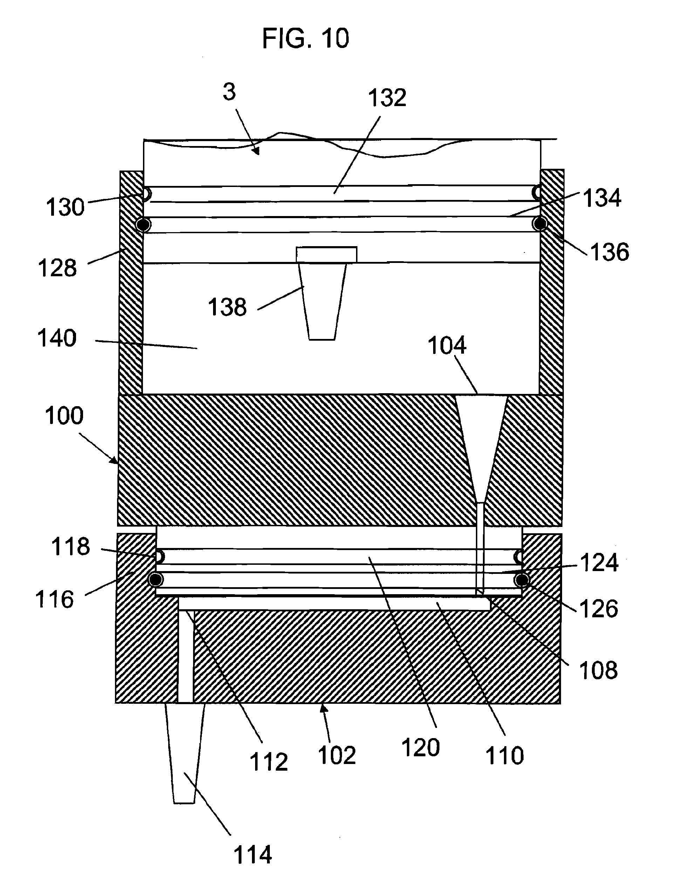

According to the variant embodiment illustrated in Fig. 10, a chamber 140 is provided between the inlet end of the inlet part 100 of the outflow regulator 4 and the outlet end of the drip chamber 3. The provision of this chamber 140 allows also the inlet hole 104 of the inlet part 100, as well as the outlet hole 108, to be formed in an eccentric position. In this way, both the inlet 104 and outlet 108 holes can be made in a single drilling operation, the manufacturing method of the outflow regulator being consequently simplified.

The chamber 140 is further capable of housing the outlet spout 138 of the drip chamber 3. In this way, the outflow regulator 4 can be applied to any drip chamber 3.

Other types of force-fitting coupling means can be further provided, such as for example compressible gaskets, which generate an elastic clamping between the peripheral wall of the chamber 140 and the outer wall of the drip chamber 3.

The chamber 140 is similarly provided at the cup-shaped end of the embodiment of Fig. 8, but with the difference that the peripheral wall of the chamber 140 has a greater axial length.

Naturally, the principle of the invention remaining unchanged, the embodiments and construction details may vary from those described and illustrated herein merely by way of example.

## Claims

1. An infusion apparatus (I) comprising
a reservoir (1) for an infusion liquid,
a drip chamber (3) placed downstream of the reservoir (1) and connected thereto, the drip chamber (3) containing an amount of infusion liquid with a level (L),
an outflow tube (6b, 6c) which starts from an outlet of the drip chamber (3) and ends with a cannula needle (2), and
alarm means (7, 8, 9, 10) for generating an alarm signal upon variations in the liquid level (L) within the drip chamber (3) relative to at least one reference level(Lmin, Lmax),
**characterized in that** said alarm means include measure means (7,8) for providing measure data indicative of the variation in the dielectric constant of the assembly formed by the drip chamber (3) and the amount of infusion liquid contained therein upon variation in the liquid level (L).

2. Apparatus according to claim 1, wherein said measure means (7, 8) comprise at least two opposed electrodes (7), the drip chamber (3) being interposed therebetween, and are arranged to measure a capacitance value between the electrodes (7).

3. Apparatus according to claim 2, wherein the electrodes (7) extend in the direction of variation of the infusion liquid level (L) within the drip chamber (3).

4. Apparatus according to claim 2 or claim 3, wherein the electrodes (7) are formed by metal plates mounted on diametrally opposite sides outside the drip chamber (3).

5. Apparatus according to claim 4, wherein the metal plates (7) are fitted in respective mounting seats formed by a peripheral wall of the drip chamber (3).

6. Apparatus according to claim 4 or claim 5, wherein the drip chamber (3) has a curved peripheral wall, particularly a cylindrical peripheral wall, and wherein the metal plates (7) forming the electrodes have a shape corresponding to the curvature of the peripheral wall portion of the drip chamber (3) to which they are mounted.

7. Apparatus according to any of claims 2 to 6, wherein the electrodes (7) are mounted to the drip chamber (3) in a removable manner.

8. Apparatus according to any of claims 2 to 7, wherein the electrodes (7) are carried by a pincer or clip member (28) having a pair of claws (30, 31) which clamp the drip chamber (3) on opposite sides.

9. Apparatus according to any of the preceding claims, wherein said alarm means include control signal generating means (9) that are operatively connected to said measure means (7,8) and alarm signalling means (10) that are operatively connected to said control signal generating means (9), said control signal generating means (9) being arranged to generate a control signal when said measure means (7, 8) detect that the liquid level (L) within the drip chamber (3) is above or below said at least one reference value (Lmin, Lmax), and to send the control signal to said alarm signalling means (10) for generating an alarm signal.

10. Apparatus according to claim 9, wherein said control signal generating means (9) include comparing means for comparing a measure signal supplied by said measure means (8) with at least one threshold value to be set, which corresponds to said at least one reference level (Lmin, Lmax).

11. Apparatus according to any of the preceding claims, wherein said alarm means include sound and/or light alarm signalling devices (10).

12. Apparatus according to claim 8, wherein said alarm means include at least one light signaller (10) mounted on one of the claws (30, 31) of the pincer or clip member (28).

13. Apparatus according to any of the preceding claims, wherein said alarm means further include a switch (14) arranged in an alarm-call activating circuit associated to a bed in a medical structure or hospital.

14. Apparatus according to claim 13, wherein the alarm-call activating circuit comprises a call push-button (13) and wherein the switch (14) is arranged in parallel to said call push-button (13).

15. Apparatus according to claim 8 and claim 9, wherein said measure means (8) and said control signal generating means (9) are housed in said pincer or clip member (28).

16. Apparatus according to claim 8, wherein at least one supply battery (35) is housed in said pincer or clip member (28).

17. Apparatus according to claim 9, wherein said control signal generating means (9) are provided with radio or infrared transmission means (11) for transmitting the control signals and wherein said alarm signalling means (10) are provided with receiving means (12) for receiving the control signals.

18. Apparatus according to claim 9, wherein said control signal generating means (9) are provided with memory means for storing an identification code for the apparatus and are arranged to send, along with each control signal, also the identification code of the respective apparatus, and wherein the alarm signalling means (10) are provided with identification means for identifying the identification code associated to each control signal.

19. Apparatus according to any of the preceding claims, further comprising processing means for storing, either continuously or periodically, the measure data supplied by said measure means (7, 8) and calculating the speed of variation in the liquid level (L).

20. Apparatus according to any of the preceding claims, further comprising an outflow regulator (4) arranged along the outflow tube (6b, 6c) between the outlet of the drip chamber (3) and the cannula needle (2).

21. Apparatus according to claim 20, wherein the outflow regulator (4) comprises a first inlet part (100) and a second outlet part (102), which are sealingly engaged with each other and can be displaced relative to each other along a preset path.

22. Apparatus according to claim 21, wherein the first part (100) of the outflow regulator (4) has, on the side facing the second part (102), an outlet hole (108) and wherein the second part (102) of the outflow regulator (4) has, on the side facing the first part (100), a collection groove (110) to which an outlet channel (112) of the second part (102) is opened, the collection groove (110) extending parallel to the relative displacement path between the two parts (100, 102) of the outflow regulator (4) and being in a position matching the outlet hole (108) of the first part (100) throughout said relative displacement path, in such a manner that when the two parts (100, 102) of the outflow regulator (4) are displaced relative to each other, the distance between the outlet hole (108) and the outlet channel (112) is changed, and the outflow rate of the infusion liquid is thus adjusted.

23. Apparatus according to claim 21 or claim 22, wherein the first and second parts (100, 102) of the outflow regulator (4) are shaped as a circular-base cylinder and engage with each other by means of snap-engaging means (118, 122; 124, 126) so as to be capable of rotating relative to each other about the central axis of the cylinder.

24. Apparatus according to claim 23, wherein said snap-engaging means (118, 122; 124, 126) are formed by a continuous circular ring (116) that is provided on a part (100; 102) of the outflow regulator (4) and by a cylindrical extension (122) that is provided on the other part (102; 100) of the outflow regulator (4).

25. Apparatus according to any of claims 21 to 24, wherein the outflow regulator (4) further comprises motor-driven means arranged to control the relative displacement of said first and second parts (100, 102), said motor-driven means being remotely operable, either manually or automatically, based on the measure data supplied by said measure means (7, 8).

26. Apparatus according to any of claims 21 to 25, wherein the first part (100) of the outflow regulator (4) is removably coupled to the drip chamber (3).

27. Apparatus according to any of claims 21 to 25, wherein the first part (100) of the outflow regulator (4) is coupled to the drip chamber (3) by form-fitting means and snap-locking means.

28. A system for monitoring a group of infusion apparatuses (I) according to claim 18, comprising a central monitoring unit (10) arranged to receive control signals generated by each of the infusion apparatuses (I), to recognize the identification code associated with each control signal and to generate an alarm signal indicating, each time, which infusion apparatus (I) has sent the control signal.
